# EUROPEAN PATENT APPLICATION

(11) **EP 3 384 910 A1**
(43) Date of publication of application: **10.10.2018**
(21) Application number: 16870277.7
(22) Date of filing: 27.09.2016
(51) Int. Cl.: A61K 31/485, A61K 9/70, A61K 47/02, A61K 47/30, A61K 47/32, A61P 17/04

(54) **TRANSDERMAL PATCH**

(30) Priority: 04.12.2015 JP 2015237950
(71) Applicant: Nichiban Co., Ltd., Bunkyo-ku Tokyo 112-8663 (JP)
(72) Inventor: TESHIMA, Emiko, Tokyo 112-8663 (JP)
(74) Representative: Graf von Stosch Patentanwaltsgesellschaft mbH
(86) International application number: PCT/JP2016/078542
(87) International publication number: WO 2017/094337

(57) **Abstract**

A patch including:
a support; and
an adhesive layer including Nalfurafine and being on the support;
wherein the adhesive layer includes
(a) an elastomer,
(b) one or more tackifiers selected from the group consisting of terpene resins and rosin resins,
(c) Nalfurafine, a salt thereof, or both thereof, and
(d) a demineralizing agent.

## Description

### Technical Field

The present invention relates to a transdermal patch.

### Background Art

Nalfurafine, which is a κ-opioid receptor agonistic compound, has been known as a compound used for improving pruritus in hemodialysis patients and improving pruritus in chronic liver disease patients.

Although the pruritus in the hemodialysis patients and the chronic liver disease patients is not a life-threatening complication, it is a non-neglectable complication in keeping the quality of life of the hemodialysis patients because the pruritus interferes with the daily life, for example, the pruritus frequently occurs during sleep to thereby cause a sleep disorder.

Among drug administration methods, an oral drug is the most common method. However, an oral agent of Nalfurafine has been recognized to cause side effects such as sleeplessness, constipation, and sleepiness. Because incidence rates of these side effects are increased in a dose-dependent manner, a transient increase of a Nalfurafine concentration in blood has been believed to possibly cause the side effects. Therefore, there has been proposed a patch as a dosage form of Nalfurafine for suppressing the transient increase of the drug concentration in blood which causes the side effects and for keeping the drug concentration in blood at a constant level for a long period of time (e.g., see PTL 1).

As for the patch of Nalfurafine, there has been proposed a patch which has an improved stability of Nalfurafine in a formulation (e.g., see PTLs 2 to 4).

However, a target to which the formulation is applied is the skin of the hemodialysis patients or the chronic liver disease patients, the skin being dry and sensitive to external stimuli. Therefore, it is believed that irritation to the skin of an application site on which the patch has been applied is preferably as low as possible. However, it has not been considered in any proposals to reduce skin irritation.

### Citation List

### Patent Literature

PTL 1 Japanese Patent Application Laid-Open (JP-A) No. 2013-147459
PTL 2 JP-A No. 2014-196279
PTL 3 International Publication No. WO/2015/025766
PTL 4 International Publication No. WO/2015/025767

### Summary of Invention

### Technical Problem

An object of the present invention is to provide an adhesive tape type patch which contains Nalfurafine as an active ingredient, which has a significantly improved transdermal absorbability of Nalfurafine, and which is less irritant to the skin.

### Solution to Problem

A patch of the present invention as a means for solving the above problems includes:
a support; and
an adhesive layer including Nalfurafine and being on the support;
wherein the adhesive layer includes
(a) an elastomer,
(b) one or more tackifiers selected from the group consisting of terpene resins and rosin resins,
(c) Nalfurafine, a salt thereof, or both thereof, and
(d) a demineralizing agent.

### Advantageous Effects of the Invention

According to the present invention, this can solve the above existing problems and achieve the above object. That is, it is possible to provide an adhesive tape type patch which contains Nalfurafine as an active ingredient, which has a significantly improved transdermal absorbability of Nalfurafine, and which is less irritant to the skin.

### Description of Embodiments

### (Patch)

A patch of the present invention includes a support and at least an adhesive layer on the support and, if necessary, further includes other layers.

### <Support>

A material of the support is not particularly limited and may be appropriately selected depending on the intended purpose. Examples thereof include paper and resins.

Examples of the paper include impregnated paper, coated paper, pure paper, kraft paper, Japanese paper, and glassine paper.

Examples of the resins include polyesters such as polyethylene terephthalate, polytrimethylene terephthalate, polybutylene terephthalate, polyethylene naphthalate, and polybutylene naphthalate; polyolefins such as polyethylene and polypropylene; polyvinyl chloride; polycarbonate; and polyurethane.

A structure of the support may be a monolayer structure or a multilayer structure. The support may be porous, network structural, foam, non-woven fabric, woven fabric, or knitted fabric.

A shape of the support is not particularly limited and may be appropriately selected depending on the intended purpose. For example, the support may be film-like, sheet-like, or plate-like.

Examples of the film-like support include plastic films such as polyester films, polyethylene films, polypropylene films, polyvinyl chloride films, polycarbonate films, and polyurethane films.

A size of the support is not particularly limited and may be appropriately selected depending on the intended purpose.

### <Adhesive layer>

The adhesive layer includes (a) an elastomer, (b) one or more tackifiers selected from the group consisting of terpene resins and rosin resins, (c) Nalfurafine, a salt thereof, or both thereof, and (d) a demineralizing agent; and, if necessary, further includes other ingredients.

A structure of the adhesive layer may be a monolayer structure or a multilayer structure.

A size of the adhesive layer is not particularly limited and may be appropriately selected depending on the intended purpose.

### <<(a) Elastomer>>

The elastomer is not particularly limited and may be appropriately selected depending on the intended purpose, but is preferably a styrene-isoprene-styrene copolymer (SIS) from the viewpoint of being able to achieve both of improvement of skin permeability and decrease of skin irritation.

Examples of the copolymer include block polymers, graft polymers, and random polymers. However, a styrene-isoprene-styrene block copolymer (SIS) is preferred because this copolymer can form intermolecular pseudo crosslinking in polystylene phases (polyaromatic vinyl compound block phases) at a normal temperature to thereby impart high cohesive force to the adhesive layer.

The elastomer may be appropriately synthesized or a commercially available product. Examples of the commercially available product include SIS 5002 (available from JSR Corporation) and Quintac 3520 (available from Zeon Corporation) which are the styrene-isoprene-styrene block copolymer.

The elastomer may be used alone or in combination.

An amount of the elastomer contained in the adhesive layer is not particularly limited and may be appropriately selected depending on the intended purpose, but is preferably 15% by mass or more, more preferably 20% by mass or more, further preferably 30% by mass or more from the viewpoint of stickiness, skin permeability, and skin irritation. Meanwhile, it is preferably 60% by mass or less, more preferably 50% by mass or less, further preferably 40% by mass or less from the viewpoint of skin permeability and skin irritation.

### <<(b) Tackifier>>

The tackifier is not particularly limited, as long as it improves adhesiveness. However, one or more tackifiers selected from the group consisting of terpene resins and rosin resins are preferably included from the viewpoint of skin permeability and skin irritation. The rosin resin is particularly preferably included from the viewpoint of stability of Nalfurafine.

Examples of the terpene resin include β-pinene resins, α-pinene resins, terpene-phenol resins, aromatic-modified terpene resins, and hydrogenated terpene resins.

Examples of the rosin resin include rosin such as gum rosin, tall oil rosin, and wood rosin; modified rosin such as hydrogenated rosin, disproportionated rosin, polymerized rosin, and maleated rosin; rosin ester such as rosin glycerin ester, hydrogenated rosin ester, hydrogenated rosin glycerin ester.

The tackifier may be appropriately synthesized or a commercially available product. Examples of the commercially available product include YS RESIN PX1150N (available from YASUHARA CHEMICAL CO., LTD.) and PINECRYSTAL KE-311 (available from ARAKAWA CHEMICAL INDUSTRIES, LTD.).

One kind of the above tackifier may be used. Alternatively, two or more kinds of the above tackifiers may be used in combination. The tackifier selected from the terpene resins and the tackifier selected from the rosin resin may be combined with each other.

An amount of the tackifier contained in the adhesive layer is not particularly limited and may be appropriately selected depending on the intended purpose, but is preferably 10% by mass or more, more preferably 15% by mass or more from the viewpoint of stickiness, skin permeability, and skin irritation. Meanwhile, it is preferably 50% by mass or less, more preferably 40% by mass or less, further preferably 30% by mass or less from the viewpoint of stickiness, skin permeability, and skin irritation.

### <<(c) Nalfurafine, salt thereof, or both thereof>>

The Nalfurafine as the active ingredient may be in a free form of Nalfurafine, but is preferably incorporated in an adhesive as a Nalfurafine salt. The Nalfurafine salt is converted into the free form by means of a demineralizing agent described below.

The Nalfurafine salt is not particularly limited and may be appropriately selected depending on the intended purpose, as long as it is a pharmaceutically acceptable salt. However, the Nalfurafine salt is preferably an acid addition salt of Nalfurafine from the viewpoint of skin permeability. Examples of the acid addition salt include hydrochloride, acetate, maleate, and oxalate. Among them, Nalfurafine hydrochloride is preferred from the viewpoint of skin permeability.

The Nalfurafine salt may be used alone or in combination.

An amount of the Nalfurafine salt contained in the adhesive layer is not particularly limited and may be appropriately selected depending on the intended purpose, but is preferably 0.1% by mass or more, more preferably 0.2% by mass or more from the viewpoint of skin permeability. Meanwhile, it is preferably 3.0% by mass or less, more preferably 2.0% by mass or less, further preferably 1.0% by mass or less from the viewpoint of skin permeability and skin irritation.

When the free form of Nalfurafine is used instead of the Nalfurafine salt, the same amount as that of the Nalfurafine salt can be incorporated in the adhesive layer.

### <<(d) Demineralizing agent>>

The demineralizing agent is not particularly limited and may be appropriately selected depending on the intended purpose, as long as it converts the Nalfurafine salt into a free form thereof. However, the demineralizing agent is preferably at least one selected from the group consisting of sodium hydrogen carbonate, an amino group-containing polymer compound, and monoethanolamine from the viewpoint of skin permeability and skin irritation.

An amount of the demineralizing agent is not particularly limited and may be appropriately selected depending on the intended purpose, but is preferably 5 mol or more, more preferably 10 mol or more relative to 1 mol of the Nalfurafine salt from the viewpoint of skin irritation and skin permeability. Meanwhile, it is preferably 30 mol or less, more preferably 25 mol or less.

A method for incorporating the demineralizing agent is not particularly limited and may be appropriately selected depending on the intended purpose. The demineralizing agent may be incorporated all at once or in several times during a production process.

When the demineralizing agent is sodium hydrogen carbonate, an amount of sodium hydrogen carbonate is not particularly limited and may be appropriately selected depending on an amount of the Nalfurafine salt to be used. However, it is preferably 0.01% by mass or more, more preferably 0.1% by mass or more, further preferably 0.3% by mass or more in the adhesive layer from the viewpoint of skin permeability. Meanwhile, it is preferably 10% by mass or less, more preferably 5.0% by mass or less, further preferably 1.0% by mass or less in the adhesive layer from the viewpoint of skin permeability and skin irritation.

The amino group-containing polymer compound is not particularly limited and may be appropriately selected depending on the intended purpose, but is preferably a copolymer composed of dialkyl (methyl or ethyl) aminoalkyl (methyl or ethyl) (meth)acrylate and a monomer unit selected from alkyl (methyl, ethyl, propyl, or butyl) (meth)acrylate, monohydroxyalkyl (ethyl, propyl, or butyl) (meth)acrylate, and a combination thereof from the viewpoint of skin permeability.

Such an amino group-containing polymer compound may be appropriately synthesized or a commercially available product. Examples of the commercially available product include a methyl methacrylate-butyl methacrylate-dimethyl aminoethyl methacrylate copolymer such as EUDRAGIT (registered trademark) EPO (available from Evonik).

When the demineralizing agent is the amino group-containing polymer compound, an amount of the amino group-containing polymer compound is not particularly limited and may be appropriately selected depending on an amount of the Nalfurafine salt to be used. However, it is preferably 0.1% by mass or more, more preferably 1.0% by mass or more in the adhesive layer from the viewpoint of skin permeability. Meanwhile, it is preferably 10% by mass or less, more preferably 5.0% by mass or less in the adhesive layer from the viewpoint of skin permeability and skin irritation.

The demineralizing agent may be used alone or in combination.

### <<Other ingredients>>

In addition to the above-described ingredients, the adhesive layer of the present invention may appropriately include, for example, a softening agent, a filler, an antioxidant, a stabilizer, and a colorant, if necessary, in an appropriate amount.

The softening agent is not particularly limited and may be appropriately selected depending on the intended purpose, as long as it decreases viscosity of the entire adhesive layer and improves a wetting property. Examples thereof include petroleum-based softening agents such as liquid paraffin; and liquid rubber-based softening agents such as liquid polyisoprene, polybutene, and polyisobutylene. A plasticizer may also be used as the softening agent. Among them, liquid paraffin, liquid polyisoprene, and polybutene are preferred from the viewpoint of excellent compatibility with the elastomer and no possibility of decreasing cohesive force of the elastomer, and liquid paraffin is more preferred from the viewpoint of excellent compatibility with a polyisoprene phase in SIS.

Examples of commercially available products of the softening agent include HICALL M-352 (available from KANEDA Co., Ltd) which is liquid paraffin.

The softening agent may be used alone or in combination.

An amount of the softening agent contained in the adhesive layer is not particularly limited, but is preferably 20% by mass or more, more preferably 30% by mass or more, further preferably 40% by mass or more from the viewpoint of excellent compatibility with the elastomer and no possibility of decreasing cohesive force of the elastomer. Meanwhile, it is preferably 60% by mass or less, more preferably 50% by mass or less from the viewpoint of excellent compatibility with the elastomer and no possibility of decreasing cohesive force of the elastomer.

The antioxidant is not particularly limited and may be appropriately selected depending on the intended purpose. Examples thereof include dibutyl hydroxytoluene (BHT), butylhydroxyanisole (BHA), Vitamin C (ascorbic acid), Vitamin E (tocopherol), sodium erythorbate, propyl gallate, sodium sulfite, and sulfur dioxide.

The antioxidant may be used alone or in combination.

An average thickness of the adhesive layer is not particularly limited and may be appropriately selected depending on the intended purpose, but is preferably 5 µm to 500 µm, more preferably 10 µm to 300 µm, further preferably 20 µm to 200 µm.

### <Other layers>

Other layers of the patch of the present invention are not particularly limited and may be appropriately selected depending on the intended purpose. Examples thereof include a cover material (e.g., a carrier film, a releasing paper, and a releasing sheet) on the support.

A shape of the cover material is not particularly limited and may be appropriately selected depending on the intended purpose. For example, the cover material is film-like, sheet-like, or plate-like.

A size of the cover material is preferably equal to or more than that of the support or the adhesive layer from the viewpoint of a handling property.

The cover material may cover a surface of the support and/or the adhesive layer.

An application time of the patch is not particularly limited and may be appropriately selected depending on the intended purpose, but the patch is preferably applied on the skin for 24 hours or longer.

### (Method for producing patch)

A method for producing the patch of the present invention is not particularly limited and may be appropriately selected depending on the intended purpose. Examples thereof include a method in which an adhesive composition including an elastomer, a tackifier, a Nalfurafine salt, and a demineralizing agent is prepared and a support is coated with the adhesive composition, and a method in which a cover material is coated with the adhesive composition and a support is bonded to the cover material.

Note that, the patch of the present invention produced as described above can be stored in a package, which is made of a packaging material having high sealing and shading properties, until immediately before use.

The packaging material having high sealing and shading properties to be used for the package is not particularly limited. Materials commonly used for packaging the patch may be used. Examples of the packaging material having a high sealing property include polyolefin-based resin films such as a polyethylene film, a polypropylene film, and a polymethylpentene film; vinyl-based resin films such as a polyvinyl chloride film, a polyvinylidene chloride film, a polyvinyl alcohol film, a polystyrene film, a polyacrylonitrile film, and an ionomer film; polyester-based resin films such as a polyethylene terephthalate film; polyamide-based resin films such as a nylon film; a cellulose-based resin film such as cellophane; polycarbonate resin films; and laminated films thereof. When it is intended to improve the shading property in addition to the sealing property, packaging materials such as laminated films of, for example, the above-described resin films and laminated films thereof with aluminium and pigment-containing resin films which are the above-described resin films added with, for example, a black pigment may be used. These resin films and laminated films may be combined in a variety of combinations to thereby be used as a laminate of multilayer films.

The patch may be contained in the package made of the laminate, sealed by a known method such as heat sealing, and stored.

Especially, one layer constituting the package is preferably a layer which is unadsorbable to the Nalfurafine and a salt thereof. For example, it is preferably an oxygen-absorbing film including, for example, an ethylene-vinyl alcohol copolymer (EVOH), polyacrylonitrile, a hygroscopic resin, and an oxygen absorber. An innermost layer constituting the package is preferably a heat sealable layer. It is preferably, for example, polyethylene, an ethylene-vinyl alcohol copolymer (EVOH), and polyacrylonitrile.

Examples of the hygroscopic resin used for a packaging material containing the hygroscopic resin include "MOISTCATCH (trade name)" (available from Kyodo Printing Co., Ltd.).

Examples of an oxygen-absorbing film used for a packaging material containing the oxygen absorber include "OXYGUARD (trade name)" (available from Toyo Seikan Co., Ltd.) and "OXYCATCH (trade name)" (available from Kyodo Printing Co., Ltd.). The oxygen absorber may be not only contained in the package (packaging material) but also separately enclosed in the package.

Moreover, it is particularly preferred from the viewpoint of stability of Nalfurafine that the patch is contained in the package and then the atmosphere within the package is replaced with and filled with nitrogen by means of, for example, a vacuum packaging machine. The patch is contained in the package to thereby produce a patch product.

### Examples

Examples of the present invention will now be described, but the present invention is not limited thereto in any way.

### (Example 1)

An adhesive composition was prepared by adding 7.97% by mass of a styrene-isoprene-styrene block copolymer (SIS; "SIS 5002 (trade name)", available from JSR Corporation) and 23.9% by mass of a styrene-isoprene-styrene block copolymer (SIS; "Quintac 3520 (trade name)", available from Zeon Corporation) serving as the elastomer; 19.1% by mass of a terpene resin ("YS RESIN PX1150N (trade name)", available from YASUHARA CHEMICAL CO., LTD.) serving as the tackifier; 45.58% by mass of liquid paraffin ("HICALL M-352 (trade name)", available from KANEDA Co., Ltd) serving as the softening agent; 0.25% by mass of Nalfurafine hydrochloride which had been dissolved in an appropriate amount of methanol serving as the Nalfurafine salt; and 3.2% by mass of an amino group-containing polymer compound (methyl methacrylate-butyl methacrylate-dimethyl aminoethyl methacrylate copolymer; "EUDRAGIT EPO (trade name)", available from Evonik) serving as the demineralizing agent to an organic solvent (toluene) so as to have a solid content of 50% by mass and mixing to homogeneous. Then, a siliconized polyester film (average thickness: 75 µm) serving as a cover material was coated with the resultant adhesive composition so as to have an average coating thickness of 30 µm. Thus, an adhesive layer was formed. A polyester film serving as a support was laminated onto the adhesive layer to thereby produce a patch 1.

The resultant patch 1 was subjected to an in vitro skin permeability test and a rabbit skin primary irritation test as described below to thereby be evaluated for skin permeability and skin irritation. Evaluation results are presented in Table 1.

### <in vitro skin permeability test method>

A diffusion cell test method was adopted as a skin permeability test. Skin permeability of a drug from the patch was tested as described below. The abdominal skin of each of hairless mice (male, 7 weeks old) was removed under pentobarbital anesthesia and mounted on a horizontal diffusion cell having a diameter of 10 mm. The inside of the cell was kept at a constant temperature condition by circulating warm water at 32°C in a double structured cell. A test patch, which had been punched to a diameter of 9 mm, was applied on a corneum side of the skin. As a receiver solution, 20% by mass aqueous polyethylene glycol 400 solution was used. Samples were taken over time in a volume of 0.5 mL at each timepoint. Each of the samples was added with 0.5 mL of methanol, mixed together, and then centrifuged to deproteinate. The thus-deproteinated solution was quantified by high performance liquid chromatography (HPLC) to thereby determine a drug concentration. Thus, a cumulative skin permeation amount for 48 hours from the start was determined. After each of the samples was taken, the receiver solution was added with 20% by mass aqueous polyethylene glycol 400 solution in an amount equal to that of the sample.

### <Rabbit skin primary irritation test>

One week before application of a test patch, the back of each of rabbits (male, about 20 weeks old) was shaved with electric hair clippers and depilated with depilatory cream. A test patch which had been punched to a diameter of 15 mm was applied. The entire application site was covered with a piece of gauze and fixed with an adhesive bandage (ELASTOPORE, available from NICHIBAN Co., Ltd.) in order to prevent the test patch from peeling off. Twenty-four hours after the application, the sample was removed. Skin reactions were visually observed 1 hour and 24 hours after the removal. The skin reactions were evaluated in accordance with Draize's evaluation criteria. That is, all of scores at 1 hour and 24 hours after the removal were summed and divided by 2 to calculate an average score for each animal. Then, an average score of all animals was calculated for each test patch, which was determined as a primary irritation index (P. I. I.).

### Draize evaluation criteria

A: Erythema and eschar formation
   Score 0: No erythema
   Score 1: Very slight erythema (barely perceptible)
   Score 2: Well-drained erythema
   Score 3: Moderate to severe erythema
   Score 4: Severe erythema (beet red) to slight eschar formation (injuries in depth)
B: Edema Formation
   Score 0: No edema
   Score 1: Very slight edema (barely perceptible)
   Score 2: Slight edema (edges of the area being distinguishable with clear raising)
   Score 3: Moderate edema (raised 1 mm or more)
   Score 4: Severe edema (raised 1 mm or more and extending beyond the area of exposure)

Skin irritation was evaluated according to the following safety categories. Safety categories of skin primary irritation index
Less than 2: Mild irritant
2 or more but less than 5: Moderate irritant
5 or more: Severe irritant

### (Example 2)

A patch 2 was produced and evaluated in the same manner as in Example 1, except that 0.84% by mass of sodium hydrogen carbonate was used as the demineralizing agent instead of 3.2% by mass of the amino group-containing polymer compound "EUDRAGIT EPO."

### (Example 3)

A patch 3 was produced and evaluated in the same manner as in Example 1, except that 19.1% by mass of a rosin resin "PINECRYSTAL KE-311 (super light colored rosin ester)" (available from ARAKAWA CHEMICAL INDUSTRIES, LTD.) was used as the tackifier instead of 19.1% by mass of the terpene resin "YS RESIN PX1150N."

### (Example 4)

A patch 4 was produced and evaluated in the same manner as in Example 3, except that 0.84% by mass of sodium hydrogen carbonate was used as the demineralizing agent instead of 3.2% by mass of the amino group-containing polymer compound "EUDRAGIT EPO."

### (Comparative Example 1)

A patch 5 was produced and evaluated in the same manner as in Example 1, except that the demineralizing agent was not incorporated and that the amount of the softening agent was changed from 45.58% by mass to 48.78% by mass.

### (Comparative Example 2)

A patch 6 was produced and evaluated in the same manner as in Example 1, except that an adhesive composition was prepared by mixing to homogeneous 69.83% by mass of an amino group-containing polymer compound (methyl methacrylate-butyl methacrylate-dimethyl aminoethyl methacrylate copolymer; "EUDRAGIT EPO (trade name)", available from Evonik) serving as the demineralizing agent; 29.92% by mass of dioctyl sebacate serving as the softening agent; and 0.25% by mass of Nalfurafine hydrochloride which had been dissolved in an appropriate amount of methanol serving as the Nalfurafine salt.

### (Comparative Example 3)

A patch 7 was produced and evaluated in the same manner as in Example 1, except that an adhesive composition was prepared by stirring to homogeneous 7.88% by mass of polyisobutylene (PIB; available from BASF, "OPPANOL B100 (trade name)") and 18.38% by mass of a styrene-isoprene-styrene block copolymer (SIS; available from JSR, "SIS 5002 (trade name)") serving as the elastomer; 57.77% by mass of an alicyclic tackifier (available from ARAKAWA CHEMICAL INDUSTRIES, LTD., "ARKON P100 (trade name)"); 15.70% by mass of liquid paraffin ("HICALL M-352 (trade name)", available from KANEDA Co., Ltd) serving as the softening agent; 0.25% by mass of Nalfurafine hydrochloride which had been dissolved in an appropriate amount of methanol serving as the Nalfurafine salt; and 0.02% by mass of sodium hydroxide serving as the demineralizing agent.

### (Comparative Example 4)

A patch 8 was produced and evaluated in the same manner as in Example 1, except that a free form of Nalfurafine was added instead of the Nalfurafine hydrochloride, that the demineralizing agent was not added, and that the amount of the softening agent was changed from 45.58% by mass to 48.78% by mass.

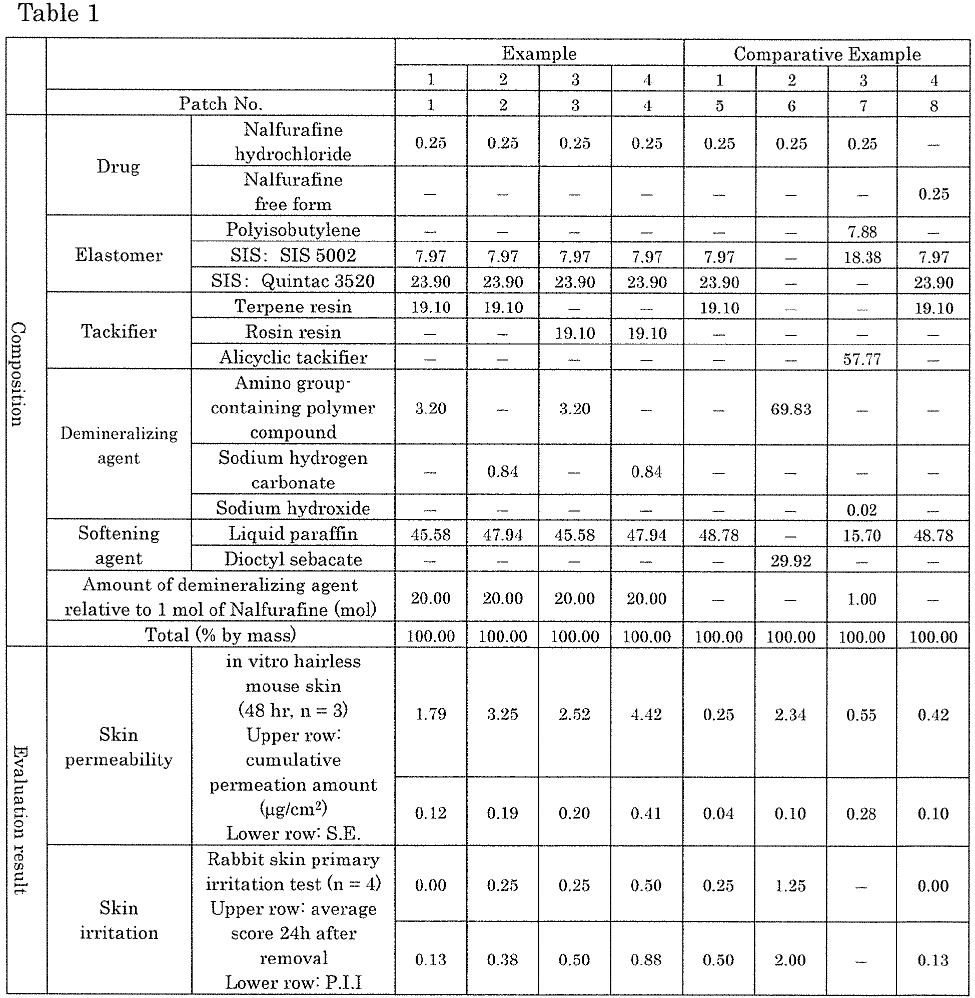

It can be seen from the results of Table 1 that the patches 1 to 4 of the present invention (Examples 1 to 4) have compositions achieving both of very high skin permeability and low skin irritation. In contrast, it can be seen that the patch 5 (Comparative Example 1) has a low skin permeation rate due to inclusion of only the hydrochloride, the patch 6 (Comparative Example 2) is irritant to the skin, and the patches 7 and 8 (Comparative Examples 3 and 4) have a low skin permeation rate.

The patches 1 to 4 (Examples 1 to 4) of the present invention have a particularly prominent effect of achieving both of high skin permeability and low skin irritation at higher levels compared to the patches 5 to 8 (Comparative Examples 1 to 4).

Aspects of the present invention are, for example, as follows:
<1> A patch including:
   a support; and
   an adhesive layer including Nalfurafine and being on the support;
   wherein the adhesive layer includes
   (a) an elastomer,
   (b) one or more tackifiers selected from the group consisting of terpene resins and rosin resins,
   (c) Nalfurafine, a salt thereof, or both thereof, and
   (d) a demineralizing agent.
<2> The patch according to <1>, wherein the elastomer is a styrene -isoprene -styrene copolymer.
<3> The patch according to <2>, wherein the styrene-isoprene-styrene copolymer is a styrene-isoprene-styrene block copolymer.
<4> The patch according to any one of <1> to <3>, wherein the demineralizing agent is at least one selected from the group consisting of sodium hydrogen carbonate and an amino group-containing polymer compound.
<5> The patch according to <4>, wherein the amino group-containing polymer compound is a methyl (meth)acrylate-butyl (meth)acrylate-dimethyl aminoethyl (meth)acrylate copolymer.
<6> The patch according to any one of <1> to <5>, wherein an amount of the demineralizing agent is 5 mol to 30 mol relative to 1 mol of the Nalfurafine salt.
<7> The patch according to any one of <1> to <6>, wherein the patch further includes a softening agent.
<8> The patch according to <7>, wherein the softening agent is liquid paraffin.
<9> The patch according to any one of <1> to <8>, wherein an amount of the elastomer included in the adhesive layer in is 15% by mass to 60% by mass,
   wherein an amount of the tackifier included in the adhesive layer is 10% by mass to 50% by mass,
   wherein an amount of the Nalfurafine salt included in the adhesive layer is 0.1% by mass to 3% by mass, and
   wherein an amount of the demineralizing agent included in the adhesive layer is 0.01% by mass to 10% by mass.
<10> The patch according to any one of <1> to <9>, wherein an amount of the elastomer included in the adhesive layer is 20% by mass to 50% by mass,
   wherein an amount of the tackifier included in the adhesive layer is 15% by mass to 40% by mass,
   wherein an amount of the Nalfurafine salt is included in the adhesive layer is 0.2% by mass to 2% by mass, and
   wherein an amount of the demineralizing agent included in the adhesive layer is 0.1% by mass to 5.0% by mass.
<11> A method for producing a patch, the method including:
   preparing an adhesive composition including:
      (a) an elastomer,
      (b) one or more tackifiers selected from the group consisting of terpene resins and rosin resins,
      (c) a Nalfurafine salt, and
      (d) a demineralizing agent; and
   forming the adhesive composition on a support.
<12> A method for producing a patch, the method including:
   preparing an adhesive composition including:
      (a) an elastomer,
      (b) one or more tackifiers selected from the group consisting of terpene resins and rosin resins,
      (c) a Nalfurafine salt, and
      (d) a demineralizing agent; and
   coating a support with the adhesive composition.
<13> A method for producing a patch, the method including:
   preparing an adhesive composition including:
      (a) an elastomer,
      (b) one or more tackifiers selected from the group consisting of terpene resins and rosin resins,
      (c) a Nalfurafine salt, and
      (d) a demineralizing agent;
   coating a cover material with the adhesive composition; and
   bonding a support to the cover material which has been coated with the adhesive composition.

## Claims

1. A patch comprising:
a support; and
an adhesive layer comprising Nalfurafine and being on the support;
wherein the adhesive layer comprises
(a) an elastomer,
(b) one or more tackifiers selected from the group consisting of terpene resins and rosin resins,
(c) Nalfurafine, a salt thereof, or both thereof, and
(d) a demineralizing agent.

2. The patch according to claim 1, wherein the elastomer is a styrene-isoprene-styrene copolymer.

3. The patch according to claim 1, wherein the demineralizing agent is at least one selected from the group consisting of sodium hydrogen carbonate and an amino group-containing polymer compound.

4. The patch according to claim 3, wherein the amino group-containing polymer compound is a methyl (meth)acrylate-butyl (meth)acrylate-dimethyl aminoethyl (meth)acrylate copolymer.

5. The patch according to any one of claims 1 to 4, wherein an amount of the demineralizing agent is 5 mol to 30 mol relative to 1 mol of the Nalfurafine salt.
